# EUROPEAN PATENT APPLICATION

(11) **EP 2 390 244 A1**
(43) Date of publication of application: **30.11.2011**
(21) Application number: 10733581.2
(22) Date of filing: 18.01.2010
(51) Int. Cl.: C07C 45/72, C07C 49/84, C07C 315/04, C07C 317/22, C08G 61/00, C07B 61/00

(54) **METHOD FOR PRODUCING CONJUGATED AROMATIC COMPOUND**

(30) Priority: 23.01.2009 JP 2009012866
(71) Applicant: Sumitomo Chemical Company, Limited, Tokyo 104-8260 (JP)
(72) Inventor: ODA, Seiji, Ibaraki-shi Osaka 567-0841 (JP); KAMIKAWA, Takashi, Nara-shi Nara 630-8442 (JP)
(74) Representative: Vossius & Partner
(86) International application number: PCT/JP2010/050858
(87) International publication number: WO 2010/084975

(57) **Abstract**

A method for producing a conjugated aromatic compound comprising reacting an aromatic compound (A) represented by the formula (1) 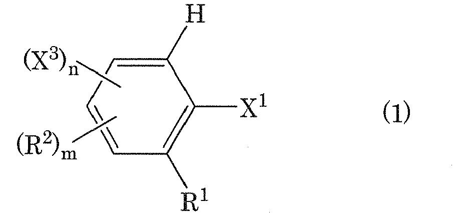 wherein n represents 0 or 1, m represents (3-n), R¹ represents a C1-C20 alkyl group etc., R² is independently in each occurrence a hydrogen atom etc., X¹ and X³ independently each represent a chlorine atom etc.,
with an aromatic compound (A) having the same structure as that of the above-mentioned aromatic compound (A) or an aromatic compound (B) wherein the aromatic compound (B) is structurally different from the above-mentioned aromatic compound (A) and one or two leaving groups selected from the group consisting of an iodine atom, a bromine atom and a chlorine atom are bonded to an aromatic ring,
in the presence of a nickel compound, a metal reducing agent, and a 2,2'-bipyridine compound having at least one electron-releasing group and having no substituent at 3-, 6-, 3'- and 6'-positions.

## Description

### Technical Field

The present invention relates to a method for producing a conjugated aromatic compound.

### Background Art

Conjugated aromatic compounds are important compounds in various fields such as agrochemicals, pharmaceuticals and electronic materials. As the method for producing it, Macromolecules 2004, 37, 4748-4754 discloses a method comprising conducting a coupling reaction of an aromatic dihalide compound in the presence of a catalytic amount of nickel chloride, triphenylphosphine, 2,2'-bipyridine and zinc.

### Disclosure of the Invention

The present invention provides:
<1> A method for producing a conjugated aromatic compound comprising reacting an aromatic compound (A) represented by the formula (1) 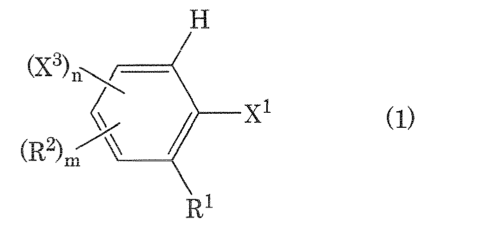 wherein n represents 0 or 1, m represents (3-n),
   R¹ represents a C1-C20 alkyl group, a C1-C20 alkoxy group or a C2-C20 acyl group, and the above-mentioned C1-C20 alkyl group, the above-mentioned C1-C20 alkoxy group and the above-mentioned C2-C20 acyl group may be substituted with at least one group selected from the group consisting of a fluorine atom, a cyano group, a C1-C20 alkoxy group, a C6-C20 aryl group, a C6-C20 aryloxy group, a C2-C20 acyl group and the formula (10): 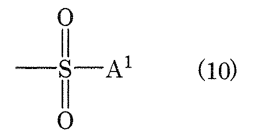 wherein A¹ represents an amino group substituted with one or two C1-C20 hydrocarbon groups, or a C1-C20 alkoxy group, and the above-mentioned hydrocarbon group and the above-mentioned alkoxy group may be substituted with at least one group selected from the group consisting of a fluorine atom, a C1-C20 alkoxy group, a C6-C20 aryl group, a C6-C20 aryloxy group, a C2-C20 acyl group, a C6-C20 arylsulfonyl group and a cyano group,
   R² is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl group, the above-mentioned C1-C20 alkoxy group, the above-mentioned C6-C20 aryl group, the above-mentioned C6-C20 aryloxy group and the above-mentioned C2-C20 acyl group may be substituted with at least one group selected from the group consisting of a fluorine atom, a cyano group, a C1-C20 alkoxy group, a C6-C20 aryl group and a C6-C20 aryloxy group, and two R²s being bonded to the neighboring carbon atoms may be bonded to form a ring, x¹ and X³ independently each represent a chlorine atom, a bromine atom or an iodine atom,
   with an aromatic compound (A) having the same structure as that of the above-mentioned aromatic compound (A) or an aromatic compound (B) wherein the aromatic compound (B) is structurally different from the above-mentioned aromatic compound (A) and one or two leaving groups selected from the group consisting of an iodine atom, a bromine atom and a chlorine atom are bonded to an aromatic ring,
   in the presence of a nickel compound, a metal reducing agent, and a 2,2'-bipyridine compound having at least one electron-releasing group and having no substituent at 3-, 6-, 3'- and 6'-positions;
<2> The method according to <1>, wherein the reaction is conducted in the presence of a 2,2'-bipyridine compound having at least two electron-releasing groups and having no substituent at 3-, 6-, 3'- and 6'-positions;
<3> The method according to <1>, wherein the 2,2'-bipyridine compound having at least one electron-releasing group and having no substituent at 3-, 6-, 3'- and 6'-positions is a bipyridine compound represented by the formula (2) 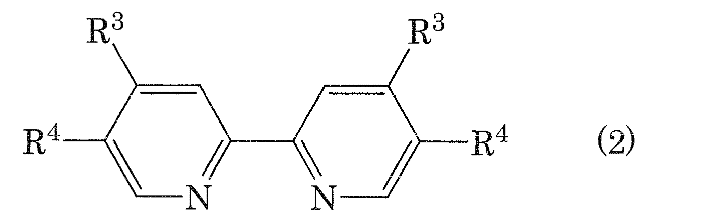 wherein R³ and R⁴ independently each represent a hydrogen atom or an electron-releasing group, with the proviso that R³ and R⁴ are not hydrogen atoms simultaneously;
<4> The method according to any one of <1> to <3>, wherein the electron-releasing group is a C1-C20 alkyl group, a C1-C20 alkoxy group, a C6-C20 aryl group or a C1-C20 dialkylamino group;
<5> The method according to any one of <1. to <4>, wherein an aromatic compound (A) is reacted with an aromatic compound (A) having the same structure as that of the aromatic compound (A);
<6> The method according to any one of <1> to <4>, wherein the aromatic compound (A) is reacted with an aromatic compound (B) being structurally different from the aromatic compound (A);
<7> The method according to <6>, wherein the aromatic compound (B) is an aromatic compound represented by the formula (3) 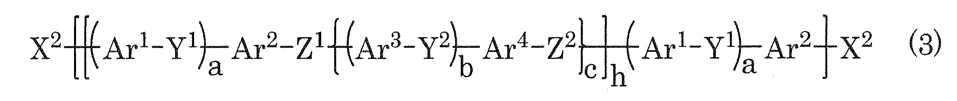 wherein a, b and c are the same or different and represent 0 or 1, and h represents an integer of 5 or more,
   Ar¹, Ar², Ar³ and Ar⁴ independently each represent a divalent aromatic group, and the divalent aromatic group may be substituted with at least one substituent selected from the group consisting of the following (a2) to (e2):
   (a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
   (b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
   (c2) a C6-C20 aryl group which may be substituted with at least one substituent selected from the group consisting of a fluorine atom, a cyano group, a C1-C20 alkoxy group and a C6-C10 aryloxy group;
   (d2) a C6-C20 aryloxy group which may be substituted with at least one substituent selected from the group consisting of a fluorine atom, a cyano group, a C1-C20 alkoxy group and a C6-C20 aryloxy group; and
   (e2) a C2-C20 acyl group which may be substituted with at least one substituent selected from the group consisting of a fluorine atom, a cyano group, a C1-C20 alkoxy group, a C6-C20 aryl group and a C6-C20 aryloxy group,
   with the proviso that (a2) and (e2) are not bonded to the neighboring carbon atoms to the carbon atoms of Ar¹ and Ar² to which X² is bonded,
   Y¹ and Y² independently each represent a single bond, -CO-, -SO₂-, -C(CH₃)₂-, -C(CF₃)₂- or a fluorene-9, 9-diyl group,
   Z¹ and Z² independently each represent -O- or -S-, and X² represents a chlorine atom, a bromine atom or an iodine atom, is used;
<8> The method according to any one of <1> to <7>, wherein the nickel compound is a nickel halide;
<9> The method according to any one of <1> to <7>, wherein the nickel compound is bis(cyclooctadiene)nickel(0);
<10> The method according to any one of <1> to <9>, wherein the metal reducing agent is zinc;
<11> The method according to any one of <1> to <10>, wherein n is 1.

### Best Mode for Carrying Out the Present Invention

The aromatic compound (A) is an aromatic compound represented by the formula (1) 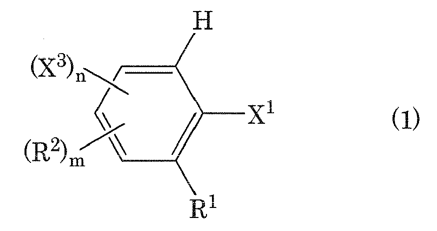 wherein n represents 0 or 1, m represents (3-n),
R¹ represents a C1-C20 alkyl group, a C1-C20 alkoxy group or a C2-C20 acyl group, and the above-mentioned Cl-C20 alkyl group, the above-mentioned C1-C20 alkoxy group and the above-mentioned C2-C20 acyl group may be substituted with at least one group selected from the group consisting of a fluorine atom, a cyano group, a C1-C20 alkoxy group, a C6-C20 aryl group, a C6-C20 aryloxy group, a C2-C20 acyl group and the formula (10): 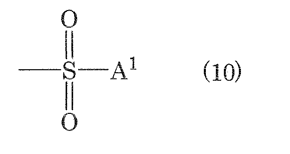 wherein A¹ represents an amino group substituted with one or two C1-C20 hydrocarbon groups, or a C1-C20 alkoxy group, and the above-mentioned hydrocarbon group and the above-mentioned alkoxy group may be substituted with at least one group selected from the group consisting of a fluorine atom, a C1-C20 alkoxy group, a C6-C20 aryl group, a C6-C20 aryloxy group, a C2-C20 acyl group, a C6-C20 arylsulfonyl group and a cyano group,
R² is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl group, the above-mentioned C1-C20 alkoxy group, the above-mentioned C6-C20 aryl group, the above-mentioned C6-C20 aryloxy group and the above-mentioned C2-C20 acyl group may be substituted with at least one group selected from the group consisting of a fluorine atom, a cyano group, a C1-C20 alkoxy group, a C6-C20 aryl group and a C6-C20 aryloxy group, and two R²s being bonded to the neighboring carbon atoms may be bonded to form a ring, X¹ and X³ independently each represent a chlorine atom, a bromine atom or an iodine atom. R¹ is bonded to one carbon atom among two carbon atoms neighboring to the carbon atom to which X¹ is bonded and a hydrogen atom is bonded to the other carbon atom.

The aromatic compound (B) compounds are compounds wherein they have at least one aromatic ring and one or two leaving groups selected from the group consisting of an iodine atom, a bromine atom and a chlorine atom are bonded to an aromatic ring. The aromatic compound (B) is structurally different from the aromatic compound (A).

Hereinafter, the aromatic compounds (A) and (B) are sometimes collectively described as the aromatic compound.

In the formula (1), R¹ represents a C1-C20 alkyl group, a C1-C20 alkoxy group or a C2-C20 acyl group.

Examples of the C1-C20 alkyl group include a C1-C20 linear, branched chain or cyclic alkyl group such as a methyl group, an ethyl group, a propyl group, an isopropyl group, a butyl group, an isobutyl group, a sec-butyl group, a tert-butyl group, a pentyl group, a 2,2-dimethylpropyl group, a hexyl group, a cyclohexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group and an icosyl group.

Examples of the C1-C20 alkoxy group include a C1-C20 linear, branched chain or cyclic alkoxy group such as a methoxy group, an ethoxy group, a propoxy group, an isopropoxy group, a butoxy group, a sec-butoxy group, a tert-butoxy group, a pentyloxy group, a 2,2-dimethylpropoxy group, a hexyloxy group, a cyclohexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group and an icosyloxy group, and a C1-C6 alkoxy group is preferable and a C1-C6 linear or branched chain alkoxy group is more preferable.

Examples of the C2-C20 acyl group include a C2-C20 aliphatic or aromatic acyl group such as an acetyl group, a propionyl group, a butyryl group, an isobutyryl group, a benzoyl group, a 1-naphthoyl group and a 2-naphthoyl group.

The above-mentioned C1-C20 alkyl group, the above-mentioned C1-C20 alkoxy group and the above-mentioned C2-C20 acyl group may be substituted with at least one substituent selected from the group consisting of a fluorine atom, a cyano group, a C1-C20 alkoxy group, a C6-C20 aryl group, a C6-C20 aryloxy group, a C2-C20 acyl group and the group represented by the above-mentioned formula (10). Examples of the C1-C20 alkoxy group and the C2-C20 aryl group include the same as described above. Examples of the C6-C20 aryl group include a phenyl group, a 4-methylphenyl group, a 2-methylphenyl group, a 1-naphthyl group, a 2-naphthyl group, a 3-phenanthryl group and a 2-anthryl group. Examples of the C6-C20 aryloxy group include those composed of the above-mentioned C6-C20 aryl group and an oxygen atom such as a phenoxy group, a 4-methylphenoxy group, a 2-methylphenoxy group, a 1-naphthyloxy group, a 2-naphthyloxy group, a 3-phenanthryloxy group and a 2-anthryloxy group.

Examples of the C1-C20 hydrocarbon group in the formula (10) include a C1-C20 linear, branched chain or cyclic alkyl group such as a methyl group, an ethyl group, a propyl group, an isopropyl group, a butyl group, an isobutyl group, a sec-butyl group, a tert-butyl group, a pentyl group, a 2,2-dimethylpropyl group, a hexyl group, a cyclohexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group and an icosyl group; a C6-C20 aryl group such as a phenyl group; a C4-C20 alkadienyl group such as a 1,3-butadiene-1,4-diyl group; a C1-C20 alkanediyl group such as a butane-1,4-diyl group and a pentane-1,5-diyl group; and a C6-C20 arylene group such as a biphenyl-2,2'-diyl group and an o-xylylene group. Examples of the amino group substituted with one or two C1-C20 hydrocarbon groups include a methylamino group a dimethylamino group, an ethylamino group, a diethylamino group, a propylamino group, a dipropylamino group, an isopropylamino group, a diisopropylamino group, a butylamino group, a dibutylamino group, a sec-butylamino group, a di-sec-butylamino group, a tert-butylamino group, a di-tert-butylamino group, a pentylamino group, a 2,2-dimethylpropylamino group, a hexylamino group, a cyclohexylamino group, a heptylamino group, an octylamino group, a nonylamino group, a decylamino group, an undecylamino group, a dodecylamino group, a tridecylamino group, a tetradecylamino group, a pentadecylamino group, a hexadecylamino group, a heptadecylamino group, an octadecylamino group, a nonadecylamino group, an icosylamino group, a pyrrolyl group, a pyrrolidinyl group, a piperidinyl group, a carbazolyl group, a dihydroindolyl group and a dihydroisoindolyl group.

Examples of the C1-C20 alkoxy group, the C6-C20 aryl group, the C6-C20 aryloxy group and the C2-C20 acyl group in the formula (10) include the same as described above, and examples of the C6-C20 arylsulfonyl group include a phenylsulfonyl group and a p-toluenesulfonyl group.

As the group represented by the formula (10), A¹ is preferably an isopropoxy group, a 2,2-dimethypropoxy group, a cyclohexyloxy group, a diethylamino group or a dodecylamino group, and A¹ is more preferably an isopropoxy group, a 2,2-dimethylpropoxy group or a cyclohexyloxy group.

R¹ is preferably a C1-C20 unsubstituted alkyl group, a C1-C20 alkyl group substituted with one or more fluorine atoms such as a trifluoromethyl group, a C1-C20 alkyl group substituted with a C1-C20 alkoxy group such as a methoxymethyl group, a C1-C20 alkyl group substituted with a cyano group such as a cyanomethyl group, a C1-C20 unsubstituted alkoxy group, a C2-C20 unsubstituted acyl group or a C2-C20 acyl group substituted with a C6-C20 aryloxy group such as a phenoxybenzoyl group.

In the formula (1), R² is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group. Examples of the C1-C20 alkyl group, the C1-C20 alkoxy group, the C6-C20 aryl group, the C6-C20 aryloxy group and the C2-C20 acyl group include the same as described above. The C1-C20 alkyl group, the C1-C20 alkoxy group, the C6-C20 aryl group, the C6-C20 aryloxy group and the C2-C20 acyl group may be substituted with at least one group selected from the group consisting of a fluorine atom, a cyano group, a C1-C20 alkoxy group, a C6-C20 aryl group and a C6-C20 aryloxy group. Alternatively, two R²s being bonded to the neighboring carbon atoms may be bonded to form a ring.

x¹ and X³ independently each represent a chlorine atom, a bromine atom or an iodine atom. X¹ and X³ are preferably the same.

In the formula (1), when n is 1 and two R²s are not hydrogen atoms, X³ is preferably not bonded to the carbon atom neighboring to the carbon atom to which R² which is not a hydrogen atom is bonded.

Examples of the aromatic compound (A) include 2-chlorotoluene, 2-ethylchlorobenzene, 2-isobutylchlorobenzene, 2-hexylchlorobenzene, 2,3-dimethoxychlorobenzene, 2,4-dimethoxychlorobenzene, 2-ethoxychlorobenzene, 2,4-dichlorotoluene, 2,5-dichlorotoluene, 2,4-dibromotoluene, 2,5-dibromotoluene, 2,4-diiodotoluene, 2,5-diiodotoluene, 2,4-dichloro-1-(trifluoromethyl)benzene, 2,5-dichloro-1-ethylbenzene, 2,5-dichloro-1-(2,2-dimethylpropyl)benzene, 2,5-dichloro-1-octylbenzene, 1,3-dichloro-4-methoxybenzene, l,4-dichloro-2-methoxybenzene, 1,3-dibromo-2-methoxybenzene, 1,3-diiodo-4-methoxybenzene, 1,4-diiodo-2-methoxybenzene, 1,4-dichloro-2-ethoxybenzene, l,4-dichloro-2-(2,2-dimethylpropyloxy)benzene, 1,4-dichloro-2-octyloxybenzene, 2,5-dichloro-1-decyloxybenzene, 4-chloroacetophenone, 2-chloroacetophenone, 4-chloropropiophenone, 1-(4-chlorophenyl)-2,2-dimethylpropanone, (4-chlorobenzoyl)cyclohexane, 4-chlorobenzophenone, p-chlorobenzalacetone, 2',4'-dichloroacetophenone, 2',5'-dichloroacetophenone, 2',4'-dibromoacetophenone, 2',5'-dibromoacetophenone, 2,5-dichlorobenzophenone and 2,5-dichloro-4'-phenoxybenzophenone.

As the aromatic compound (A), a commercially available one may be used, and one produced according to the known method may be used.

Examples of the aromatic ring of the aromatic compound (B) include an aromatic hydrocarbon ring such as a benzene ring, a biphenyl ring, a naphthalene ring, a fluorene ring, an anthracene ring and a phenanthrene ring, and a heteroaromatic ring such as a thiophene ring, a pyrrole ring and a pyridine ring.

The aromatic ring may be substituted with at least one group uninvolved in the reaction, and specific examples of the group uninvolved in the reaction include the following (a1) to (jl).
(a1) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b1) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c1) a C6-C20 aryl group which may be substituted with at least one substituent selected from the group consisting of a fluorine atom, a cyano group, a C1-C20 alkoxy group and a C6-C20 aryloxy group;
(d1) a C6-C20 aryloxy group which may be substituted with at least one substituent selected from the group consisting of a fluorine atom, a cyano group, a C1-C20 alkoxy group and a C6-C20 aryloxy group;
(e1) a C2-C20 acyl group which may be substituted with at least one substituent selected from the group consisting of a fluorine atom, a cyano group, a C1-C20 alkoxy group, a C6-C20 aryl group and a C6-C20 aryloxy group;
(f1) a C2-C20 acyloxy group which may be substituted with at least one substituent selected from the group consisting of a fluorine atom, a cyano group, a C1-C20 alkoxy group, a C6-C20 aryl group and a C6-C20 aryloxy group;
(g1) a C6-C20 arylsulfonyl group which may be substituted with at least one substituent selected from the group consisting of a fluorine atom, a cyano group, a C1-C20 alkoxy group, a C6-C20 aryl group and a C6-C20 aryloxy group;
(h1) the group represented by the above-mentioned formula (10);
(i1) a cyano group;
(j1) a fluorine atom.

Examples of the C1-C20 alkoxy group in the above-mentioned (a1) to (g1) include the same as described above, and a C1-C6 alkoxy group is preferable.

Examples of the C6-C20 aryl group and the C6-C20 aryloxy group in the above-mentioned (a1) to (g1) include the same as described above, respectively.

Examples of the C1-C20 alkyl group in the above-mentioned (a1) include the same as described above.

Examples of the C2-C20 acyl group in the above-mentioned (e1) include the same as described above.

Examples of the C2-C20 acyloxy group in the above-mentioned (f1) include those composed of the above-mentioned C2-C20 acyl group and an oxygen atom such as an acetyloxy group, a propionyloxy group, a butyryloxy group, an isobutyryloxy group, a benzoyloxy group, a 1-naphthoyloxy group and a 2-naphthoyloxy group.

Examples of the C6-C20 arylsulfonyl group in the above-mentioned (g1) include the same as described above.

As (a1) a C1-C20 unsubstituted alkyl group, a C1-C20 alkyl group substituted with one or more fluorine atoms such as a trifluoromethyl group, a C1-C20 alkyl group substituted with a C1-C20 alkoxy group such as a methoxymethyl group, and a C1-C20 alkyl group substituted with a cyano group such as a cyanomethyl group are preferable.

As (b1) a C1-C20 unsubstituted alkoxy group and a C1-C20 alkoxy group substituted with a C1-C20 alkoxy group such as a methoxymethoxy group are preferable.

As (c1), a C6-C20 unsubstituted aryl group is preferable.

As (d1), a C6-C20 unsubstituted aryloxy group is preferable.

As (e1) a C2-C20 unsubstituted acyl group and a C2-C20 acyl group substituted with a C6-C20 aryloxy group such as a phenoxybenzoyl group are preferable.

As (f1) a C2-C20 unsubstituted acyloxy group and a C2-C20 acyloxy group substituted with a C6-C20 aryloxy group such as a phenoxybenzoyloxy group are preferable.

As (g1), a C6-C20 unsubstituted arylsulfonyl group is preferable.

As (h1), a group represented by the formula (10) wherein A¹ is an isopropoxy group, a 2,2-dimethylpropyl group, a cyclohexyloxy group, a diethylamino group or a dodecylamino group is preferable, and a group represented by the formula (10) wherein A¹ is an isopropoxy group, a 2,2-dimethylpropyl group or a cyclohexyloxy group is more preferable.

The leaving group is preferably a chlorine atom or a bromine atom.

Examples of the aromatic compound (B) include the same as the aromatic compound (A) .

Alternatively, specific examples of the aromatic compound (B) also include an aromatic compound represented by the formula (3) 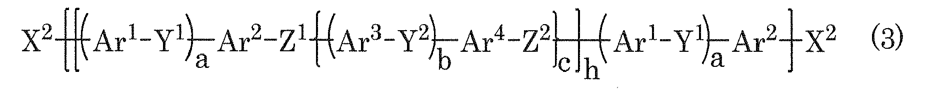 wherein a, b and c are the same or different and represent 0 or 1, and h represents an integer of 5 or more,
Ar¹, Ar², Ar³ and Ar⁴ independently each represent a divalent aromatic group, and the divalent aromatic group may be substituted with at least one substituent selected from the group consisting of the following (a2) to (e2):
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group,
with the proviso that (a2) and (e2) are not bonded to the neighboring carbon atoms to the carbon atoms of Ar¹ and Ar² to which X² is bonded,
Y¹ and Y² independently each represent a single bond, -CO-, -SO₂-, -C(CH₃)₂-, -C(CF₃)₂- or a fluorene-9, 9-diyl group,
Z¹ and Z² independently each represent -O- or -S-, and X² represents a chlorine atom, a bromine atom or an iodine atom.

In the formula (3), h is preferably an integer of 10 or more.

Examples of the divalent aromatic group in Ar², Ar³, Ar⁴ and Ar⁵ include a divalent monocyclic aromatic group such as a 1,3-phenylene group, a 1,4-pherlylene group and 4,4'-biphenyl-1,1'-diyl group; a divalent condensed aromatic group such as a naphthalene-1,3-diyl group, a naphthalene-1,4-diyl group, a naphthalene-1,5-diyl group, a naphthalene-1,6-diyl group, a naphthalene-1,7-diyl group, a naphthalene-2,6-diyl group, a naphthalene-2,7-diyl group and a 9H-fluorene-2,7-diyl group; and a divalent heteroaromatic group such as a pyridine-2,5-diyl group, a pyridine-2,6-diyl group, a quinoxaline-2,6-diyl group, a thiophene-2,5-diyl group, 2,2'-bithiophene-5,5'-diyl group, a pyrrole-2,5-diyl group, a 2,2'-bipyridine-5,5'-diyl group, a pyrimidine-2,5-diyl group, a quinoline-5,8-diyl group, a quinoline-2,6-diyl group, an isoquinoline-1,4-diyl group, an isoquinoline-5,8-diyl group, 2,1,3-benzothiadiazole-4,7-diyl group, a benzimidazole-4,7-diyl group, a quinoxaline-5,8-diyl group and a quinoxaline-2,6-diyl group. Among them, preferred are the divalent monocyclic aromatic group and the divalent condensed aromatic group, and more preferred are a 1,4-phenylene group, a naphthalene-1,4-diyl group, a naphthalene-1,5-diyl group, a naphthalene-2,6-diyl group and a naphthalene-2,7-diyl group.

The divalent aromatic group may be substituted with at least one substituent selected from the group consisting of the following (a2) to (e2) .
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group.

Examples of the C1-C20 alkoxy group, the C6-C20 aryl group, the C6-C20 aryloxy group, the C1-C20 alkyl group and the C2-C20 acyl group in (a2) to (e2) include the same as described above.

Examples of (a2) include the same as the above-mentioned (a1). Examples of (b2) include the same as the above-mentioned (b1). Examples of (c2) include the same as the above-mentioned (c1). Examples of (d2) include the same as the above-mentioned (d1). Examples of (e2) include the same as the above-mentioned (e1).

As X², a chlorine atom and a bromine atom are preferable.

Specific examples of the aromatic compound represented by the formula (3) include the compounds represented by the following and compounds wherein both terminal chlorine atoms in the compounds represented by the following are replaced by bromine atoms. In the following formulae, h represents the same meanings as the above.

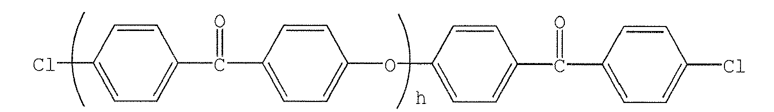

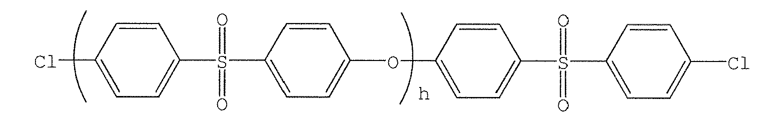

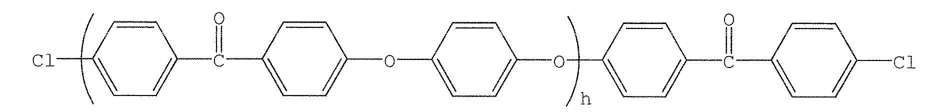

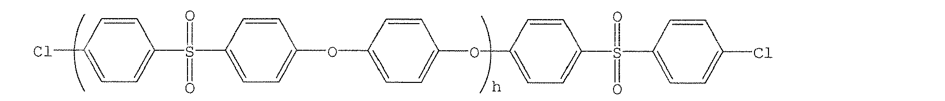

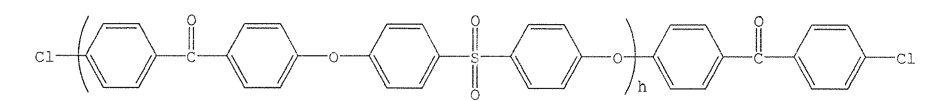

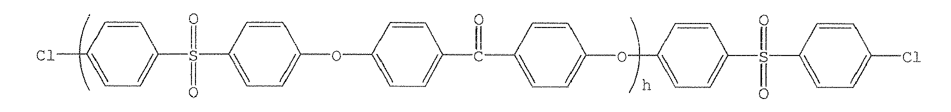

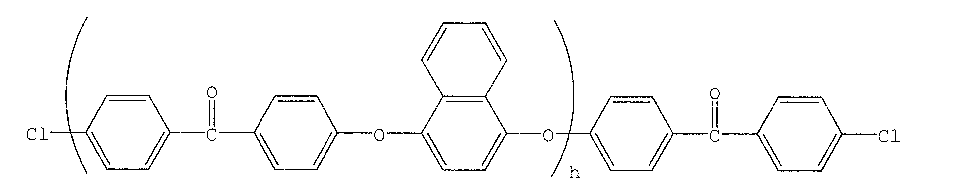

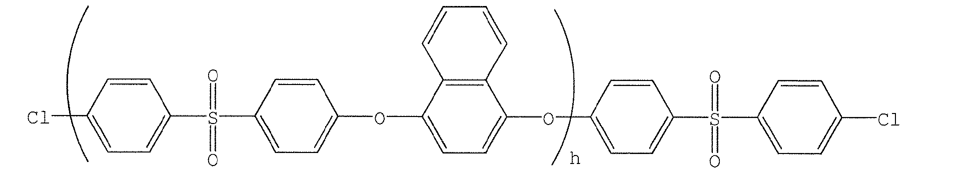

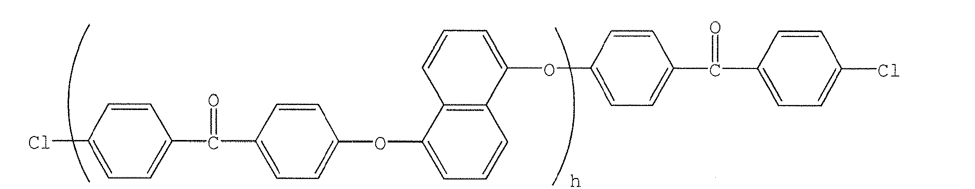

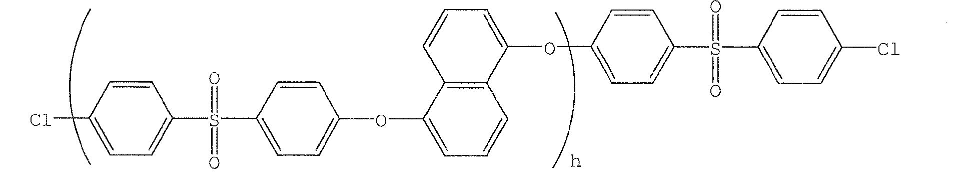

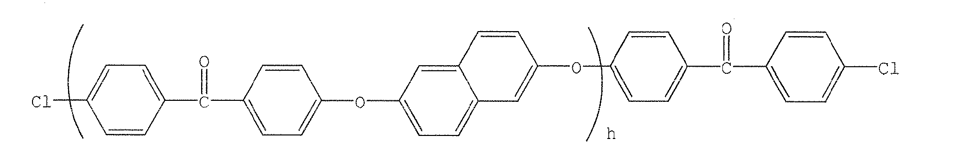

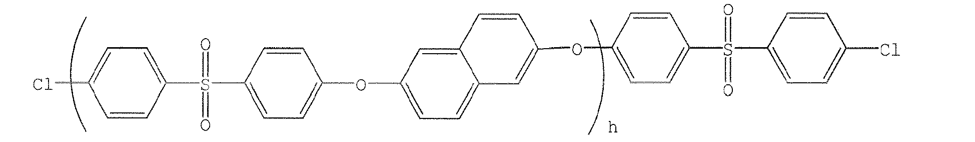

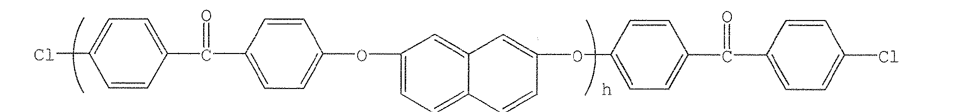

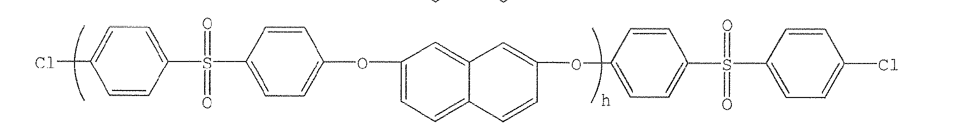

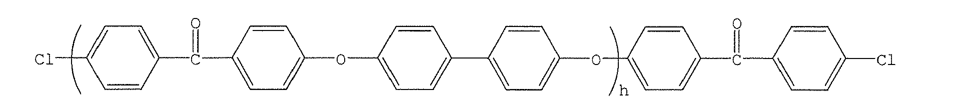

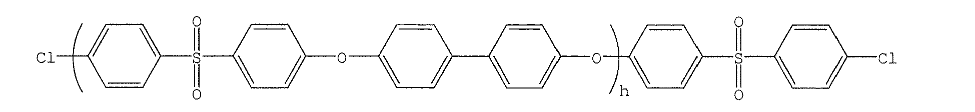

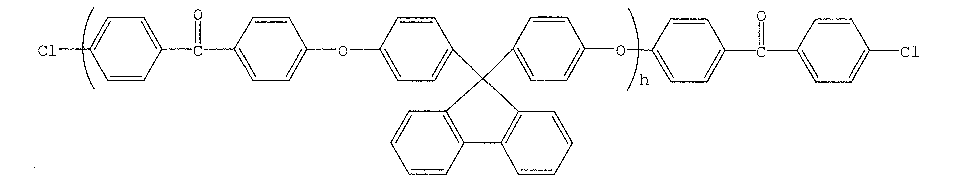

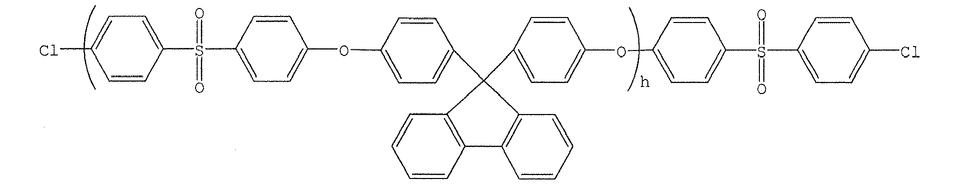

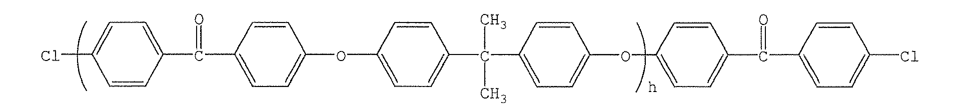

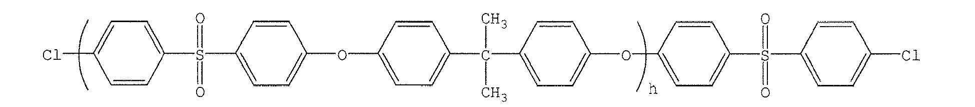

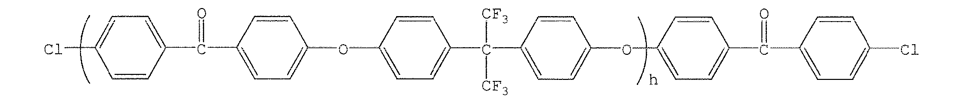

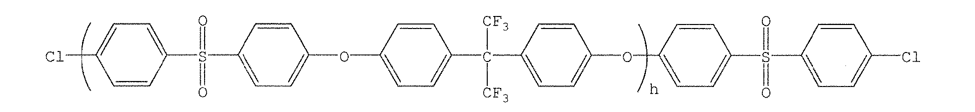

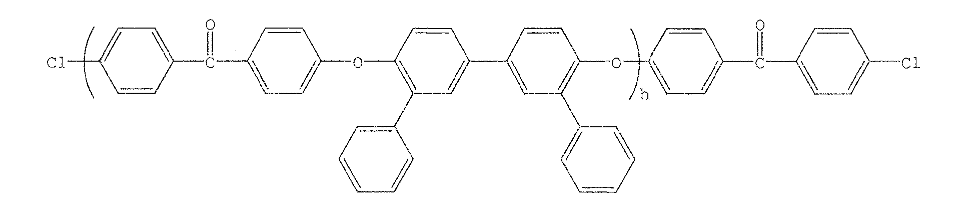

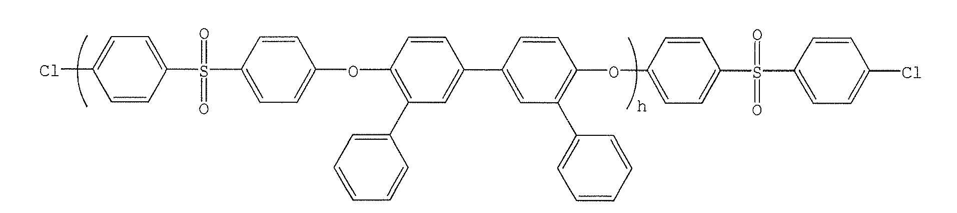

As the aromatic compound represented by the formula (3), one produced according to known methods such as JP Patent No. 2,745,727 may be used and a commercially available one may be used. Examples of the commercially available one include SUMIKA EXCEL PES manufactured by Sumitomo Chemical Company, Limited.

As the aromatic compound represented by the formula (3), one having a weight average molecular weight equivalent to polystyrene of 2,000 or more is preferably used, and one having a weight average molecular weight equivalent to polystyrene of 3,000 or more is more preferable.

The present invention is a method for producing a conjugated aromatic compound comprising reacting the aromatic compound (A) with the aromatic compound (A) having the same structure as that of the above-mentioned aromatic compound (A) or the aromatic compound (B) being structurally different from the above-mentioned aromatic compound (A) in the presence of a nickel compound, a metal reducing agent, and a 2,2`-bipyridine compound having at least one electron-releasing group and having no substituent at 3-, 6-, 3'- and 6'-positions.

Specific examples of cases where the aromatic compound (A) is reacted with the aromatic compound (B) being structurally different from the above-mentioned aromatic compound. (A) include a case where the aromatic compound represented by the formula (10) and being structurally different from the above-mentioned aromatic compound (A) is used as the aromatic compound (B); and a case where the aromatic compound represented by the formula (3) is used as the aromatic compound (B).

When the aromatic compound (A) is reacted with the aromatic compound (B) being structurally different from the above-mentioned aromatic compound (A), the content of the repeating unit derived from the aromatic compound (A) and the content of the repeating unit derived from the aromatic compound (B) in the conjugated aromatic compound obtained can be adjusted, respectively, by adjusting the used amount of the aromatic compound (A) and the used amount of the aromatic compound (B) respectively.

Examples of the nickel compound include a zero-valent nickel compound such as bis(cyclooctadiene)nickel(0) and tetrakis(triphenylphosphine)nickel(0); and a divalent nickel compound such as a nickel halide (for example, nickel fluoride, nickel chloride, nickel bromide, nickel iodide and the like), a nickel carboxylate (for example, nickel formate, nickel acetate and the like), nickel sulfate, nickel carbonate, nickel nitrate, nickel acetylacetonate and (dimethoxyethane)nickel chloride, and bis(cyclooctadiene)nickel(0) and a nickel halide are preferable.

While the used amount of the nickel compound may be a catalytic amount, and when the used amount thereof is too small, the yield of a conjugated aromatic compound tends to be low or a conjugated aromatic compound having a small molecular weight tends to be obtained, and when the used amount thereof is too much, the isolation procedure of an conjugated aromatic compound after completion of reaction tends to be cumbersome, and therefore, the used amount of the nickel compound is usually 0.001 to 0.8 mole and preferably 0.01 to 0.3 mole per 1 mole of all of the aromatic compounds involved in the reaction.

"Metal reducing agent" means a metal capable of reducing divalent nickel to zero-valent nickel. Specific examples thereof include zinc, magnesium, manganese, aluminum and sodium, and zinc, magnesium and manganese are preferable, and zinc is more preferable. Usually, a commercially available metal reducing agent is used. Alternatively, powdery or chip-type metal reducing agent is usually used. The used amount of the metal reducing agent is usually 1 mole or more per 1 mole of all of the aromatic compounds involved in the reaction. While the upper limit thereof is not limited, when the used amount thereof is too much, the isolation procedure of an conjugated aromatic compound after completion of reaction tends to be cumbersome and it easily becomes to be economically disadvantageous, and therefore, it is practically 10 moles or less and preferably 5 moles or less.

"Electron-releasing group" in the 2,2'-bipyridine compound having at least one electron-releasing group and having no substituent at 3-, 6-, 3'- and 6'-positions means a substituent wherein the value of a defined by Hammett formula described in Chemical Reviews 1991, 91, 165-195 is negative.

Specific examples thereof include a C1-C20 alkyl group, a C1-C20 alkoxy group, a C6-C20 aryl group and a C1-C20 dialkylamino group. Among them, preferred are a C1-C20 alkyl group and a C1-C20 alkoxy group.

Examples of the C1-C20 alkyl group include a methyl group, an ethyl group, a propyl group, an isopropyl group, a butyl group, an isobutyl group, a sec-butyl group, a tert-butyl group, a pentyl group, a 2,2-dimethylpropyl group, a cyclopentyl group, a hexyl group, a cyclohexyl group, a heptyl group, a 2-methylpentyl group, an octyl group, a 2-ethylhexyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group and an icosyl group. Preferred are a methyl group and a tert-butyl group.

Examples of the C1-C20 alkoxy group include a methoxy group, an ethoxy group, a propoxy group, an isopropoxy group, a butoxy group, a sec-butoxy group, a tert-butoxy group, a pentyloxy group, a 2,2-dimethylpropoxy group, a hexyloxy group, a cyclohexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group and an icosyloxy group. Preferred is a methoxy group.

Examples of the C6-C20 aryl group include a phenyl group, a 4-methylphenyl group, a 2-methylphenyl group, a 1-naphthyl group, a 2-naphthyl group, a 3-phenanthryl group and a 2-anthryl group. Preferred is a phenyl group.

Examples of the C1-C20 dialkylamino group include a dimethylamino group, a diethylamino group, a dipropylamino group, a diisopropylamino group, a dibutylamino group and a di(2,2-dimethylpropyl)amino group. Preferred is a dimethylamino group.

The 2,2'-bipyridine compound may be a 2,2'-bipyridine compound having at least one electron-releasing group and having no substituent at 3-, 6-, 3'- and 6'-positions, and may have a group other than the electron-releasing group at a position other than 3-, 6-, 3'- and 6'-positions.

The 2,2'-bipyridine compound preferably has at least two electron-releasing groups. The 2,2'-bipyridine compound wherein each of two pyridine rings has one electron-releasing group is preferable.

As the 2,2'-bipyridine compound having at least two electron-releasing groups and having no substituent at 3-, 6-, 3'- and 6'-positions, a bipyridine compound represented by the formula (2) 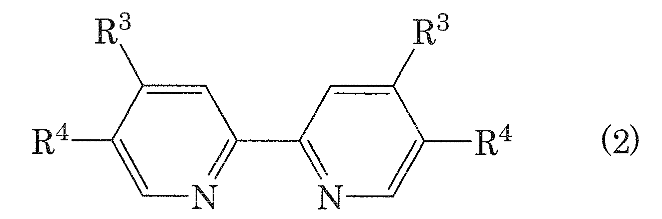 wherein R³ and R⁴ independently each represent a hydrogen atom or an electron-releasing group, with the proviso that R³ and R⁴ are not hydrogen atoms simultaneously is preferable.

Examples of the 2,2'-bipyridine compound include 4,4'-dimethyl-2,2'-bipyridine, 4,4'-dinonyl-2,2'-bipyridine, 4,4'-dimethoxy-2,2'-bipyridine, 4,4'-diphenyl-2,2'-bipyridine, 5,5'-dimethyl-2,2'-bipyridine, 4,4',5,5'-tetramethyl-2,2'-bipyridine, 4,4°-bis(1,1-dimethylethyl)-2,2'-bipyridine and 4,4'-bis(dimethylamino)-2,2'-bipyridine.

As the 2,2'-bipyridine compound, a commercially available one may be used, and one produced according to known methods such as Bull. Chem. Soc. Jpn., 63, 80-87 (1990) may be used.

Two or more kinds of the 2,2'-bipyridine compounds may be mixed to be used.

The used amount of the 2,2'-bipyridine compound is usually 0.2 to 2 moles and preferably 0.5 to 1.7 moles relative to 1 mole of the nickel compound.

The 2,2'-bipyridine compound and the nickel compound may be previously contacted to be used. The 2,2'-bipyridine compound and the nickel compound may be mixed in a solvent and the resultant mixture containing a nickel complex may be used as it is, and the nickel complex may be isolated from the mixture to be used. By change of a color of the mixture, the preparation of the nickel complex can be discerned.

The reaction of the aromatic compound (A) with the aromatic compound (A) having the same structure as that of the above-mentioned aromatic compound (A) or the aromatic compound (B) being structurally different from the above-mentioned aromatic compound (A) is usually carried out in the presence of a solvent. The solvent may be one in which the used aromatic compounds and the produced conjugated aromatic compound can be dissolved. Specific examples of the solvent include an aromatic hydrocarbon solvent such as toluene and xylene; an ether solvent such as tetrahydrofuran and 1,4-dioxane; an aprotic polar solvent such as dimethylsulfoxide, N-methyl-2-pyrrolidone, N,N-dimethylformamide, N,N-dimethylacetamide and hexamethylphosphoric triamide; and a halogenated hydrocarbon solvent such as dichloromethane and dichloroethane. These solvents may be used alone, and two or more kinds thereof may be mixed to be used. Among them, preferred are the ether solvent and the aprotic polar solvent, and more preferred are tetrahydrofuran, dimethylsulfoxide, N-methyl-2-pyrrolidone and N,N-dimethylacetamide. When the used amount of the solvent is too much, a conjugated aromatic compound having small molecular weight tends to be obtained, and when the used amount thereof is too small, the property of the reaction mixture tends to be bad, and therefore, it is usually 1 to 200 parts by weight and preferably 5 to 100 parts by weight per 1 part by weight of all of the aromatic compounds used.

The reaction is usually conducted by mixing the aromatic compounds, the nickel compound, metal reducing agent and the 2,2'-bipyridine compound in an atmosphere of an inert gas such as nitrogen gas. The mixing order is not limited.

The reaction temperature is usually 0 to 250°C and preferably 30 to 100°C. The reaction time is usually 0.5 to 48 hours.

In order to improve a reaction rate, a halogen compound may be added to the reaction system. Examples of the halogen compound include a sodium halide such as sodium fluoride, sodium chloride, sodium bromide and sodium iodide, a potassium halide such as potassium fluoride, potassium chloride, potassium bromide and potassium iodide, and a quaternary ammonium halide such as tetraethylammonium fluoride, tetraethylammonium chloride, tetraethylammonium bromide and tetraethylammonium iodide. A sodium halide is preferable, and sodium iodide is more preferable. The used amount thereof is usually 0.01 to 1 mole per 1 mole of the all of the aromatic compounds involved in the reaction, and preferably 0.05 to 0.2 mole.

The conjugated aromatic compound can be obtained by thus reaction, and "conjugated aromatic compound" means a compound having at least one aromatic ring and possessing a delocated π-electron system in a part of or all of its molecule.

When the conjugated aromatic compound produced is a polymer, for example, after completion of the reaction, the conjugated aromatic compound is precipitated by mixing a solvent in which the conjugated aromatic compound produced is not soluble or is poorly soluble with the reaction mixture, followed by separating the precipitated conjugated aromatic compound from the reaction mixture by filtration, thereby being able to isolate it. The solvent in which the conjugated aromatic compound produced is not soluble or is poorly soluble may be mixed with the reaction mixture and then an aqueous acid solution such as hydrochloric acid is added thereto followed by separating the conjugated aromatic compound precipitated from the reaction mixture by filtration. The molecular weight and structure of the conjugated aromatic compound obtained can be analyzed by a conventional means such as gel permeation chromatography and NMR. Examples of the solvent in which the produced conjugated aromatic compound is not soluble or is poorly soluble include water, methanol, ethanol and acetonitrile, and water and methanol are preferable.

When the conjugated aromatic compound produced is not a polymer, for example, after completion of the reaction, the conjugated aromatic compound produced can be isolated by concentrating the reaction mixture. The conjugated aromatic compound isolated may be further purified by a conventional purification means such as column chromatography, distillation and recrystallization.

When the aromatic compound (A) having two leaving groups is used, a conjugated aromatic compound having a repeating unit derived from the aromatic compound (A) is obtained, and the weight-average molecular weight thereof equivalent to polystyrene is usually 1,000 to 2,000,000.

When the aromatic compound (A) wherein n is 1 in the formula (1) is used, specific examples of the conjugated aromatic compound obtained include a conjugated aromatic compound comprising a repeating unit represented by the formula (4) 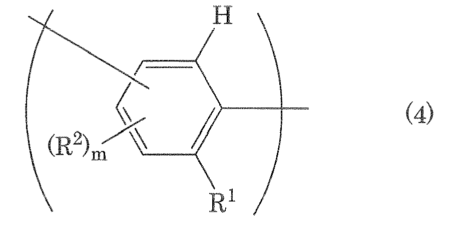 wherein R¹ and R² are the same as defined above, and it usually contains the repeating unit of 2 to 10,000, and the weight-average molecular weight thereof equivalent to polystyrene is usually 500 to 3,000,000.

When the aromatic compound (A) wherein n is 1 in the formula (1) is used and the aromatic compound represented by the formula (3) is used as the aromatic compound (B), specific examples of the conjugated aromatic compound obtained include a conjugated aromatic compound comprising a repeating unit represented by the formula (4) and the segment represented by the following formula (5) 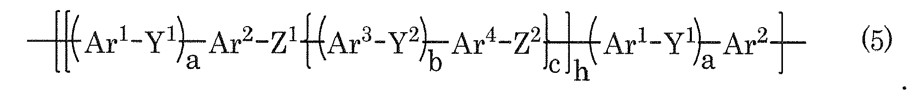 The weight-average molecular weight of the conjugated aromatic compound equivalent to polystyrene is usually 3,000 to 3,000,000.

Specific examples of the segment represented by the formula (5) include the segments represented by the following formulae (24a) to (24x). Additionally, in the following formulae, h represents the same meaning as defined above and is preferably an integer of 10 or more.

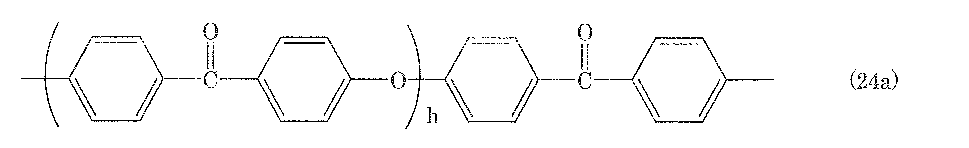

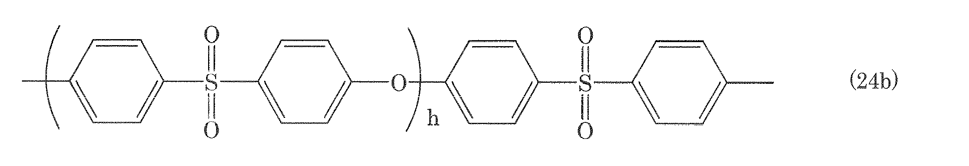

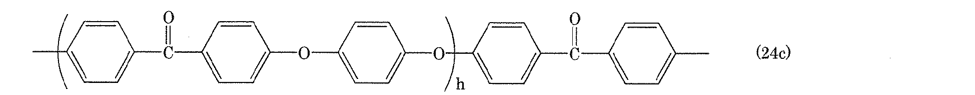

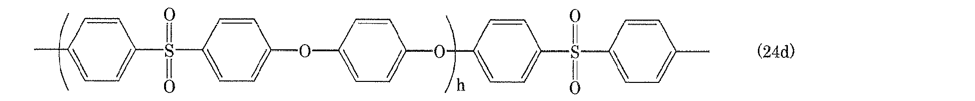

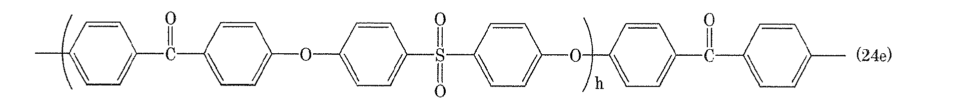

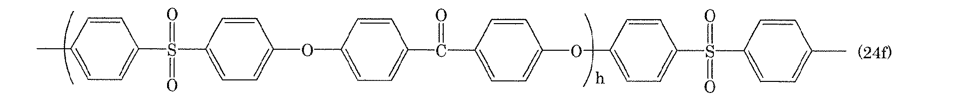

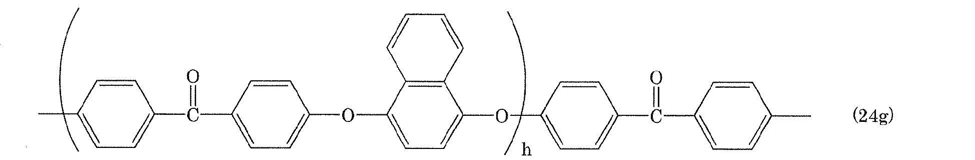

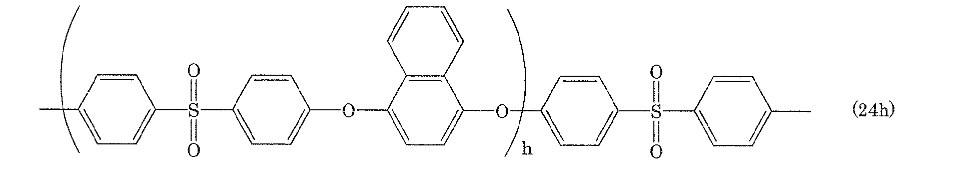

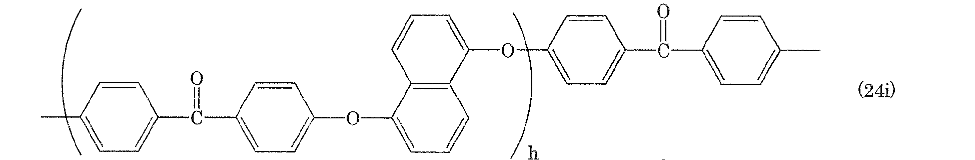

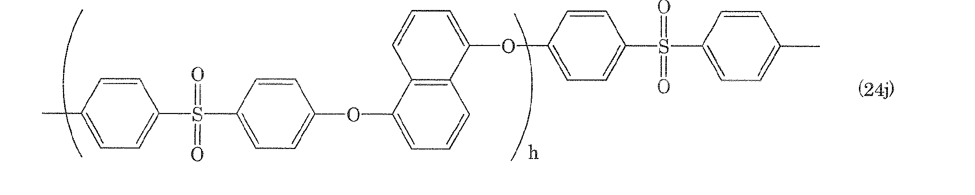

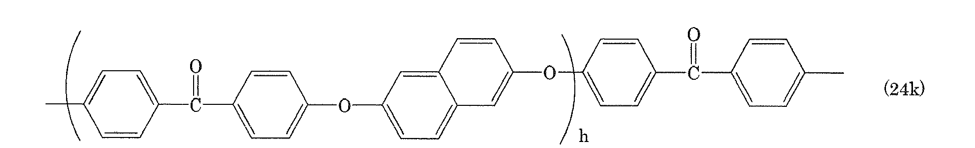

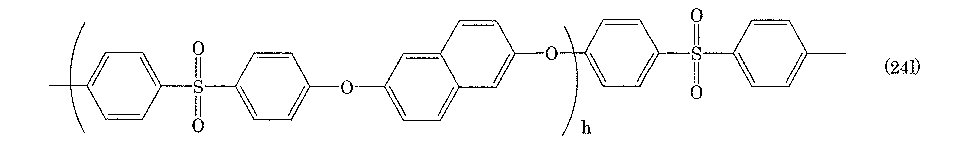

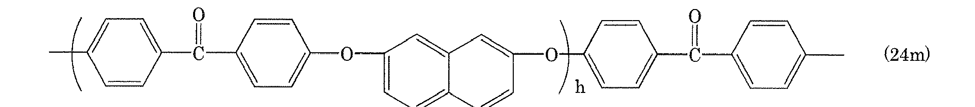

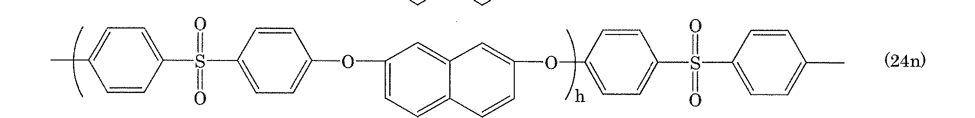

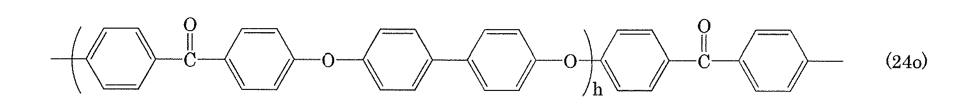

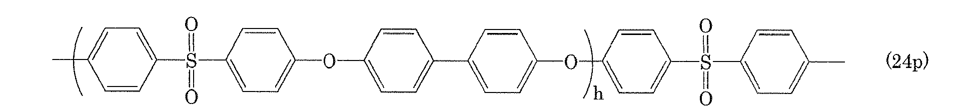

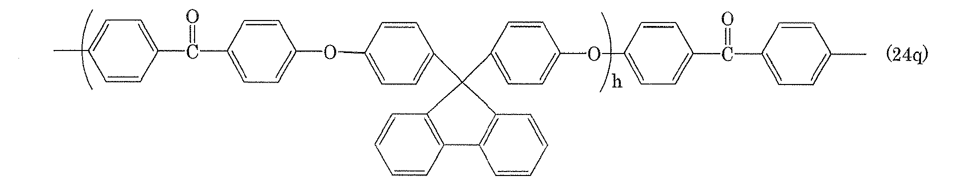

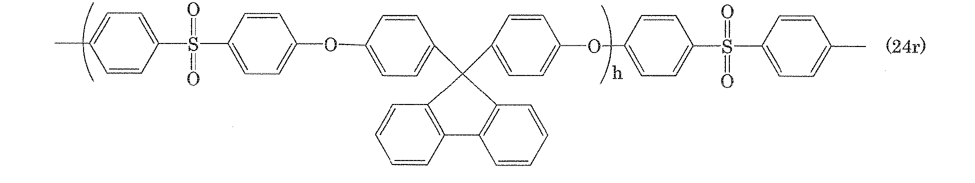

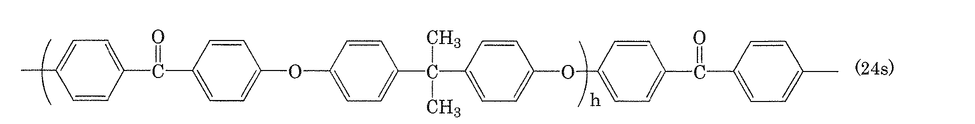

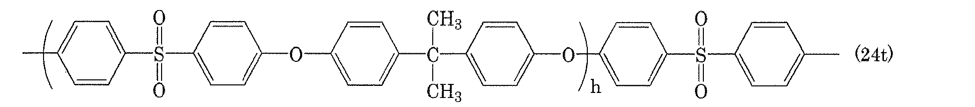

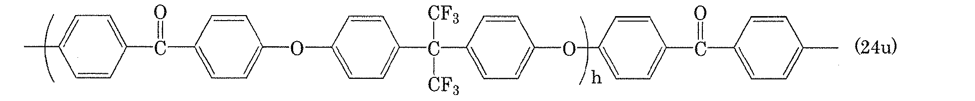

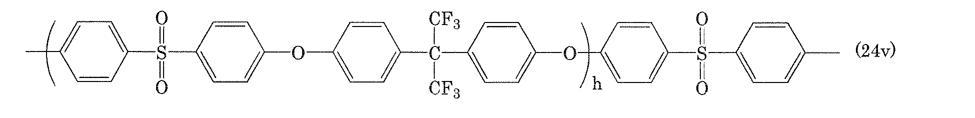

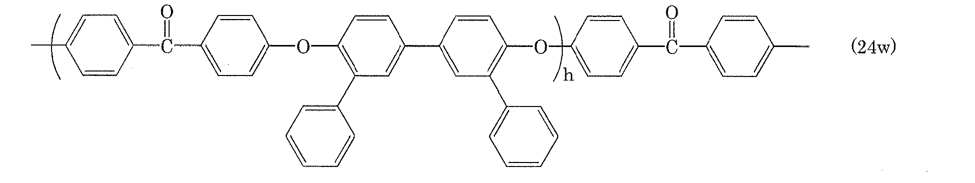

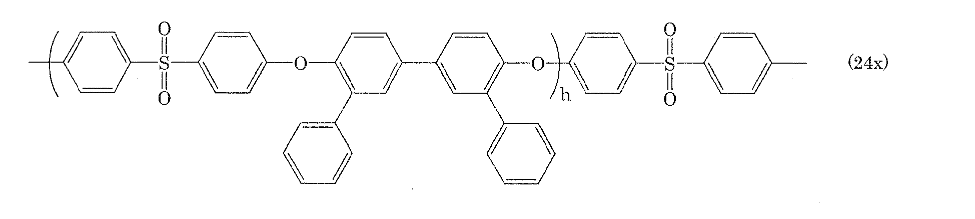

The content of each of repeating units in the conjugated aromatic compound comprising two or more kinds of the repeating units can be adjusted by arbitrarily adjusting the used amount of the aromatic compounds used.

### Examples

The present invention will be further illustrated by Examples in more detail below, but the present invention is not limited to these Examples. When the conjugated aromatic compound obtained was not a polymer, it was analyzed with gas chromatography internal standard method or liquid chromatography internal standard method, and the yield thereof was calculated from their results. When the conjugated aromatic compound obtained was a polymer, it was analyzed with gel permeation chromatography (hereinafter, simply referred to as GPC), of which analytical condition was as followed, and the weight-average molecular weight (Mw) and the number-average molecular weight (Mn) thereof equivalent to polystyrene were calculated from its result.

### <Analytical Condition>

GPC measuring apparatus: CTO-10A (manufactured by Shimadzu Corporation)
Column: TSK-GEL (manufactured by Tosoh Coporation)
Column temperature: 40°C
Eluent: N,N-dimethylacetamide containing lithium bromide (concentration of lithium bromide: 10 mmol/dm³)
Flow rate: 0.5 mL/minute
Detection wavelength: 300 nm

### [Example 1]

To a reaction container made of glass and equipped with a cooling apparatus, 31 mg of nickel bromide, 30 mg of 4,4'-dimethoxy-2,2'-bipyridine and 110 mg of zinc powder were added in an atmosphere of nitrogen at room temperature. To the mixture obtained, 240 mg of 2,5-dichloro-4'-phenoxybenzophenone and 5 mL of N, N-dimethylacetamide were added at room temperature. The reaction was conducted at 70°C for 4 hours to obtain a reaction mixture containing a conjugated aromatic compound consisting of a repeating unit represented by the following formula a (i).

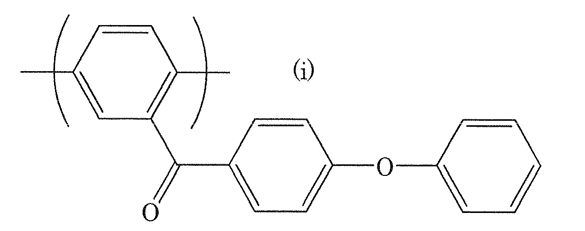

Mw of the conjugated aromatic compound was 174,000, and Mn thereof was 42,000.

### [Example 2]

The reaction was conducted according to the same manner as that of Example 1, except that 26 mg of 3,3'-dimethyl-2,2'-bipyridine was used in place of 30 mg of 4,4'-dimethoxy-2,2'-bipyridine, a reaction mixture containing a conjugated aromatic compound consisting of a repeating unit represented by the formula (i) described in Example 1 was obtained. Mw of the conjugated aromatic compound was 38,000, and Mn thereof was.16,000.

### [Example 3]

The reaction was conducted according to the same manner as that of Example 1, except that 26 mg of 4,4'-dimethyl-2,2'-bipyridine was used in place of 30 mg of 4,4'-dimethoxy-2,2'-bipyridine, a reaction mixture containing a conjugated aromatic compound consisting of a repeating unit represented by the formula (i) described in Example 1 was obtained. Mw of the conjugated aromatic compound was 31,000, and Mn thereof was 15,000.

### [Example 4]

The reaction was conducted according to the same manner as that of Example 1, except that 13 mg of 4,4'-dimethyl-2,2'-bipyridine and 15 mg of 4,4'-dimethoxy-2,2'-bipyridine were used in place of 30 mg of 4,4'-dimethoxy-2,2'-bipyridine, a reaction mixture containing a conjugated aromatic compound consisting of a repeating unit represented by the formula (i) described in Example 1 was obtained. Mw of the conjugated aromatic compound was 122,000, and Mn thereof was 37,000.

### [Comparative Example 1]

The reaction was conducted according to the same manner as that of Example 1, except that 22 mg of 2,2'-bipyridine was used in place of 30 mg of 4,4'-dimethoxy-2,2'-bipyridine, a reaction mixture containing a conjugated aromatic compound consisting of a repeating unit represented by the formula (i) described in Example 1 was obtained. Mw of the conjugated aromatic compound was 4,000, and Mn thereof was 3,000.

### [Example 5]

To a reaction container made of glass and equipped with a cooling apparatus, 31 mg of nickel bromide, 30 mg of 4,4'-dimethoxy-2,2'-bipyridine, 110 mg of zinc powder, a solution obtained by dissolving 240 mg of 2,5-dichloro-4'-phenoxybenzophenone in 3 mL of N,N-dimethylacetamide and a solution obtained by dissolving 100 mg of SUMIKA EXCEL PES 3100P represented by the following formula (ii) : 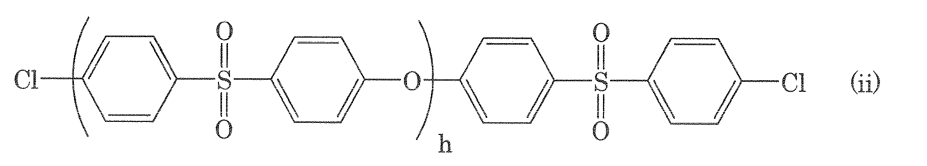 which hade been manufactured by Sumitomo Chemical Company, Limited; Mw 36, 000 and Mn 18, 000 which had been measured by the above analytical condition, in 2 mL of N,N-dimethylacetamide were added in an atmosphere of nitrogen at room temperature. Then, the polymerization reaction was conducted at 70°C for 4 hours to obtain a reaction mixture containing a conjugated aromatic compound consisting of a repeating unit represented by the above-mentioned formula (i) and a segment represented by the following formula 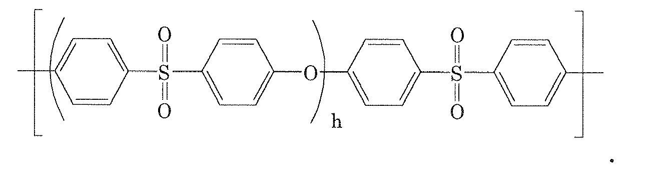 Mw of the conjugated aromatic compound was 74, 000, and Mn thereof was 23,000.

### [Example 6]

The reaction was conducted according to the same manner as that of Example 5, except that 100 mg of an aromatic compound represented by the formula (iii) 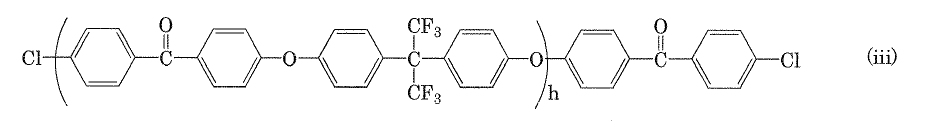 of which Mw was 6, 500 and Mn was 4,700 which had been measured by the above analytical condition, in place of 100 mg of SUMIKA EXCEL PES 3100P represented by the formula (ii), a reaction mixture containing a conjugated aromatic compound consisting of a repeating unit represented by the above-mentioned formula (i) and a segment represented by the following formula: 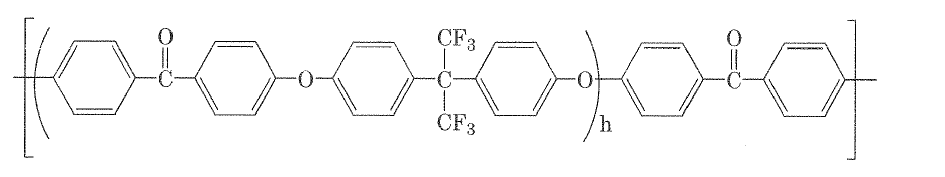 was obtained. Mw of the conjugated aromatic compound was 78, 000, and Mn thereof was 28,000.

### Industrial Applicability

According to the present invention, a conjugated aromatic compound can be produced more advantageously.

## Claims

1. A method for producing a conjugated aromatic compound comprising reacting an aromatic compound (A) represented by the formula (1) 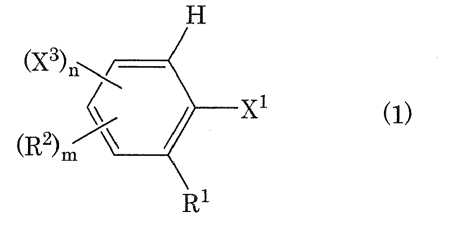 wherein n represents 0 or 1, m represents (3-n),
R¹ represents a C1-C20 alkyl group, a C1-C20 alkoxy group or a C2-C20 acyl group, and the above-mentioned C1-C20 alkyl group, the above-mentioned C1-C20 alkoxy group and the above-mentioned C2-C20 acyl group may be substituted with at least one group selected from the group consisting of a fluorine atom, a cyano group, a C1-C20 alkoxy group, a C6-C20 aryl group, a C6-C20 aryloxy group, a C2-C20 acyl group and the formula (10): 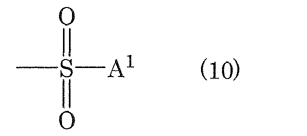 wherein A¹ represents an amino group substituted with one or two C1-C20 hydrocarbon groups, or a C1-C20 alkoxy group, and the above-mentioned hydrocarbon group and the above-mentioned alkoxy group may be substituted with at least one group selected from the group consisting of a fluorine atom, a C1-C20 alkoxy group, a C6-C20 aryl group, a C6-C20 aryloxy group, a C2-C20 acyl group, a C6-C20 arylsulfonyl group and a cyano group,
R² is independently in each occurrence a hydrogen atom, a fluorine atom, a C1-C20 alkyl group, a C1-C20 alkoxy group, a C6-C20 aryl group, a C6-C20 aryloxy group, a C2-C20 acyl group or a cyano group, and the above-mentioned C1-C20 alkyl group, the above-mentioned C1-C20 alkoxy group, the above-mentioned C6-C20 aryl group, the above-mentioned C6-C20 aryloxy group and the above-mentioned C2-C20 acyl group may be substituted with at least one group selected from the group consisting of a fluorine atom, a cyano group, a C1-C20 alkoxy group, a C6-C20 aryl group and a C6-C20 aryloxy group, and two R²s being bonded to the neighboring carbon atoms may be bonded to form a ring, x¹ and X³ independently each represent a chlorine atom, a bromine atom or an iodine atom,
with an aromatic compound (A) having the same structure as that of the above-mentioned aromatic compound (A) or an aromatic compound (B) wherein the aromatic compound (B) is structurally different from the above-mentioned aromatic compound (A) and one or two leaving groups selected from the group consisting of an iodine atom, a bromine atom and a chlorine atom are bonded to an aromatic ring,
in the presence of a nickel compound, a metal reducing agent, and a 2,2'-bipyridine compound having at least one electron-releasing group and having no substituent at 3-, 6-, 3'- and 6'-positions.

2. The method according to claim 1, wherein the reaction is conducted in the presence of a 2,2'-bipyridine compound having at least two electron-releasing groups and having no substituent at 3-, 6-, 3'- and 6'-positions.

3. The method according to claim 1, wherein the 2,2'-bipyridine compound having at least one electron-releasing group and having no substituent at 3-, 6-, 3'- and 6'-positions is a bipyridine compound represented by the formula (2) 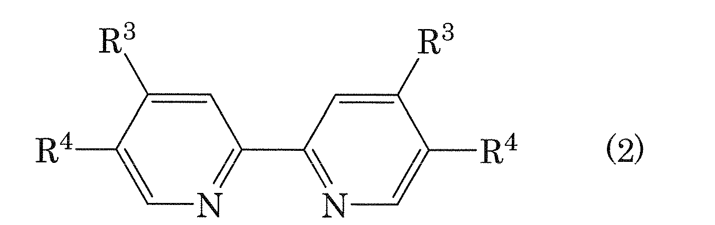 wherein R³ and R⁴ independently each represent a hydrogen atom or an electron-releasing group, with the proviso that R³ and R⁴ are not hydrogen atoms simultaneously.

4. The method according to any one of claims 1 to 3, wherein the electron-releasing group is a C1-C20 alkyl group, a C1-C20 alkoxy group, a C6-C20 aryl group or a C1-C20 dialkylamino group.

5. The method according to claim 1, wherein an aromatic compound (A) is reacted with an aromatic compound (A) having the same structure as that of the aromatic compound (A).

6. The method according to claim 1, wherein the aromatic compound (A) is reacted with an aromatic compound (B) being structurally different from the aromatic compound (A).

7. The method according to claim 6, wherein the aromatic compound (B) is an aromatic compound represented by the formula (3) 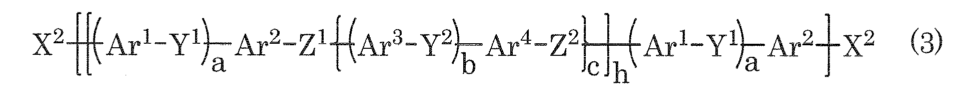 wherein a, b and c are the same or different and represent 0 or 1, and h represents an integer of 5 or more,
Ar¹,Ar², Ar³ and Ar⁴ independently each represent a divalent aromatic group, and the divalent aromatic group may be substituted with at least one substituent selected from the group consisting of the following (a2) to (e2):
(a2) a C1-C20 alkyl group which may be substituted with at least one substituent selected from the group consisting of a fluorine atom, a cyano group, a C1-C20 alkoxy group, a C6-C20 aryl group and a C6-C20 aryloxy group;
(b2) a C1-C20 alkoxy group which may be substituted with at least one substituent selected from the group consisting of a fluorine atom, a cyano group, a C1-C20 alkoxy group, a C6-C20 aryl group and a C6-C20 aryloxy group;
(c2) a C6-C20 aryl group which may be substituted with at least one substituent selected from the group consisting of a fluorine atom, a cyano group, a C1-C20 alkoxy group and a C6-C10 aryloxy group;
(d2) a C6-C20 aryloxy group which may be substituted with at least one substituent selected from the group consisting of a fluorine atom, a cyano group, a C1-C20 alkoxy group and a C6-C20 aryloxy group; and
(e2) a C2-C20 acyl group which may be substituted with at least one substituent selected from the group consisting of a fluorine atom, a cyano group, a C1-C20 alkoxy group, a C6-C20 aryl group and a C6-C20 aryloxy group,
with the proviso that (a2) and (e2) are not bonded to the neighboring carbon atoms to the carbon atoms of Ar¹ and Ar² to which X² is bonded,
Y¹ and Y² independently each represent a single bond, -CO-, -SO₂-, -C(CH₃)₂-, -C(CF₃)₂- or a fluorene-9,9-diyl group,
Z¹ and Z² independently each represent -O- or -S-, and X² represents a chlorine atom, a bromine atom or an iodine atom, is used.

8. The method according to claim 1, wherein the nickel compound is a nickel halide.

9. The method according to claim 1, wherein the nickel compound is bis(cyclooctadiene)nickel(0).

10. The method according to claim 1, wherein the metal reducing agent is zinc.

11. The method according to claim 1, wherein n is 1.
